# EUROPEAN PATENT SPECIFICATION

(11) **EP 1 137 946 B1**
(45) Date of publication and mention of the grant of the patent: **19.10.2005**
(21) Application number: 99959565.5
(22) Date of filing: 10.12.1999
(51) Int. Cl.: G01N 33/68

(54) **USE OF VASCULAR ENDOTHELIAL GROWTH FACTOR (VEGF)**
VERWENDUNG DES VASKULAREN ENDOTHELIALEN WACHSTUMSFAKTORS (VEGF)
UTILISATION DU FACTEUR DE CROISSANCE ENDOTHELIAL VASCULAIRE

(30) Priority: 11.12.1998 GB 9827407; 08.04.1999 GB 9908061
(43) Date of publication of application: 04.10.2001
(73) Proprietor: Eisai Co., Ltd., Tokyo 112-88 (JP)
(72) Inventor: STADDON, James Martin, Gower Street, London WC1E 6BT (GB); MORGAN, Mary Louise, Gower Street, London WC1E 6BT (GB)
(74) Representative: Cornish, Kristina Victoria Joy
(86) International application number: PCT/GB1999/004162
(87) International publication number: WO 2000/036421

(56) References cited:
- WO-A-98/14186
- ESSER S ET AL: "Vascular endothelial growth factor induces VE-cadherin tyrosine phosphorylation in endothelial cells." JOURNAL OF CELL SCIENCE, (1998 JUL) 111 ( PT 13) 1853-65, XP000889611
- STADDON, J.M. ET AL.: "p120, a p120-Related Protein (p100), and the Cadherin/Catenin Complex" THE JOURNAL OF CELL BIOLOGY, vol. 130, no. 2, July 1995 (1995-07), pages 369-381, XP000892283
- WONG, E.Y.M. ET AL.: "Vascular endothelial growth factor stimulates dephosphorylation of the catenins p120 and p100 in endothelial cells" BIOCHEMICAL JOURNAL, vol. 346, 15 February 2000 (2000-02-15), pages 209-216, XP000892346

## Description

The present invention relates inter alia to signalling transduction pathways and the regulation of cell-cell adhesion. Such regulation is important in a variety of pathological situations.

It is known that epithelial and endothelial cells adhere to their neighbours through specific adhesion complexes [see Cell 1996 Feb 9;84(3):345-57. Cell adhesion: the molecular basis of tissue architecture and morphogenesis. Gumbiner BM]. In particular, the adherens junction is the site of calcium-dependent adhesion between these cells. The adherens junction is composed of single-pass transmembrane adhesion molecules called the cadherins. Cadherins are calcium-dependent adhesion molecules that play a key role in initiating and maintaining intercellular contact. The classic cadherins are single-pass, transmembrane glycoproteins that interact homotypically with cadherins on neighbouring cells. Crucial for cadherin function is association via their cytoplasmic tail with a group of proteins termed catenins. Cadherin binds directly to β- or γ-catenin, proteins related to the Drosophila segment polarity gene product Armadillo, which then binds to α-catenin, a vinculin homologue, that links the complex to the actin-based cytoskeleton [see J Cell Biochem 1996 Jun 15;61(4):514-23. Cadherin-catenin complex: protein interactions and their implications for cadherin function. Aberle H, Schwartz H, Kemler R].

p120, another catenin, was originally discovered as a Src substrate, tyrosine phosphorylation of which may be involved in cellular transformation [see Bioessays 1997 Oct;19(10):883-91. Tyrosine phosphorylation and cadherin/catenin function. Daniel JM, Reynolds AB]. It too is a member of the Armadillo family and was subsequently realised to be associated with cadherins in both epithelial and endothelial cells. A p120 related protein p100 also exists, probably as a result of alternative splicing of the p120 gene. More details in respect of p104 and p120 can be found from WO95/13820, WO96/16170 and WO98/14186.

The role of the adherens junction is to initiate and maintain cell-cell contact. Furthermore, the formation of other junctions such as the tight junction is critically dependent on the prior formation and maintenance of adherens junctions. Aberrant regulation of adherens junctions can result in metastasis, loss of contact inhibition and tissue oedema [see Pathol Res Pract 1996 Jul;192(7):694-707. Regulation of the invasion suppressor function of the cadherin/catenin complex. Vermeulen S, Van Marck V, Van Hoorde L, Van Roy F, Bracke M, Mareel M.]

There is a fair amount of evidence that the cadherin/catenin complex is a target for signalling pathways. Of particular relevance to the present invention is that p120 and p100 are targets for a protein kinase C (PKC) activated signalling pathway (the PKC-p120/p100 pathway) in epithelial cells. In this case, protein kinase C activation resulted in decreased serine/threonine phosphorylation of p120/p100. Protein kinase C activation must cause p120/p120 dephosphorylation by p120/p100 kinase inhibition or activation of the corresponding phosphatase [J Biol Chem 1997 Dec 12;272(50):31894-901. Dephosphorylation of the cadherin-associated p100/p120 proteins in response to activation of protein kinase C in epithelial cells. Ratcliffe MJ, Rubin LL, Staddon JM].

The PKC-p120/p100 pathway is clearly present in endothelial cells, as revealed by the use of phorbol-12,13-dibutyrate, a pharmacological activator of PKC. Endothelial cells, possess receptors for agents such as histamine and thrombin, known to be involved in the inflammatory response. It was also found that p120/p100 phosphorylation in endothelial cells is clearly regulated by these inflammatory agents (as described in WO 98/14186).

VE-cadherin tyrosine phosphorylation has been found to be induced by vascular endothelial growth factor (VEGF) [J Cell Science 1998 111: 1853-1865 Esser S, Lampugnani, M G, Corada M, Dejana, E and Risau W].

According to a first aspect, the present invention provides the use of VEGF to screen for a substance capable of affecting the phosphorylation state of p120 and/or p100. The screen may be for a substance capable of blocking the dephosphorylation of p120 and/or p100. The substance may affect the phosphorylation state of p120 and/or p100, e.g. by blocking the dephosphorylation of p120 and/or p100. The dephosphorylation may be from the phosphorylated serine and/or threonine residues present in p120 and/or p100. Preferably the screen is to identify a substance which is capable of affecting the phosphorylation state of p120 and/or p100. A method to screen for a substance capable of affecting the phosphorylation state of p120 and/or p100, comprising the use of VEGF, is also provided.

In a second aspect, the present invention provides a substance identifiable by the use of a screen as set out in the first aspect. The substance may be identified by the ue of a screen as set out in the first aspect. Preferred features of the first aspect of the invention apply to the second aspect.

In a third aspect, the present invention provides the use of VEGF to screen for a substance capable of interfering with a VEGF-initiated pathway regulating p120/p100 serine/threonine phosphorylation. The pathway may be the PKC-p120/p100 pathway. Preferably the screen is to identify an inhibitor and/or a competitor and/or an activator of VEGF (in particular when the VEGF is involved in the pathway regulating p120/p100 serine/threonine phosphorylation).

In a fourth aspect, the present invention provides an inhibitor and/or a competitor and/or an activator of VEGF identifiable by a screen as set out in the third aspect. The inhibitor and/or competitor and/or activator may be identified by the screen. Preferred features of the third aspect of the invention also apply to the fourth aspect.

In a fifth aspect, the present invention provides a method to identify the phosphorylation state of p120 and/or p100 comprising identifying a mobility shift of these proteins as revealed by an immunoblotting procedure. The phosphorylation state of p120 and/or p100 may be affected by VEGF. The method may be for the diagnosis of a disease, which disease may involve VEGF. The method may also be used in the evaluation of efficacy of a drug being used or tested to control a disease involving VEGF.

In a sixth aspect, the present invention provides a method to identify the phosphorylation state of p120 and/or p100 comprising the use of an antibody specific for one or more of the phosphorylation sites on p120 and/or p100. The method may be for the diagnosis of a disease, which disease may involve VEGF. The method may also be used in the evaluation of efficacy of a drug being used or tested to control a disease involving VEGF.

In a seventh aspect, the present invention provides a method to screen for a compound or other agent that interferes with a signalling pathway capable of being initiated by VEGF to regulate p120/p100 phosphorylation, comprising the identification of the phosphorylation state of p120 and/or p100, in the presence of the compound or the other agent, and optionally comparing the phosphorylation state with a standard. The identification of the phosphorylation state may be by monitoring a band shift of p120 and/or p100. Preferably the screen identifies a compound or other agent that interferes with a signalling pathway capable of being initiated by VEGF.

In an eighth aspect, the present invention provides a compound or other agent that interferes with a signalling pathway capable of being initiated by VEGF, identifiable by a method as described in the seventh aspect of the present invention. Preferred features of the seventh aspect also apply to the eighth aspect. The compound or agent may interefere with a signalling pathway that is initiated by VEGF. It may be identified by a method as set out in the seventh aspect.

The present invention discloses that vascular endothelial growth factor (VEGF), a potent and important angiogenic factor, also known as vascular permeability factor [see Cancer J Sci Am 1998 Jul-Aug;4(4):209-17. New developments in angiogenesis: a major mechanism for tumor growth and target for therapy. Jones A, Harris AL; Thromb Haemost 1997 Jul;78(1):678-83. Angiogenesis in embryos and ischemic diseases. Breier G, Damert A, Plate KH, Risau W; Endocr Rev 1997 Feb;18(1):4-25. The biology of vascular endothelial growth factor. Ferrara N, Davis-Smyth T], also stimulates p120 and p100 dephosphorylation in endothelial cells. VEGF₁₆₅ as well as other variants of VEGF may be used, such as amino acid sequence variants (muteins or polymorphic variants), a species of VEGF comprising additional residues, and any naturally occurring allelic and/or splice variants [Neufeld G *et al, Cancer Metastasis Rev* 1996 Jun **15(2)** 153-8. "Similarities and differences between the vascular endothelial growth factor (VEGF) splice variants"] The p120 and p100 dephosphorylation is revealed by SDS-PAGE and immunoblotting as a band-shift (also called mobility shift), hereafter referred to as the p120/p100 band shift (as shown in Example 1). The response is rapid and blocked by an inhibitor of the VEGF receptor tyrosine kinase. Therefore, the invention provides a simple method of visualizing phosphorylation effects triggered by VEGF on p120/p100. Regulation of p120/p100 phosphorylation, as adherens junction-associated proteins may turn out to be important in their regulation of cell-cell adhesion, angiogenesis and tissue oedema. VEGF and therefore p120/p100 serine/threonine phosphorylation may be important in pathologies involving cancer and hypoxia.

VEGF is known to be an important angiogenic factor. Anti-angiogenic and therefore anti VEGF strategies are being considered as therapy for cancer and other hypoxic conditions such as retinopathy. VEGF is also a key regulator of vascular permeability in pathologies involving hypoxia. The ensuing oedema can contribute significantly to the morbidity and mortality of patients. Regulation of cadherin function may be crucial to the mode of action of VEGF. The present invention reports on a method to visualize phosphorylation effects of VEGF on the cadherin-associated proteins p120 and p100. The invention could be useful in diagnosis and screening for agents interfering with VEGF initiated signalling that regulates p120/p100 phosphorylation.

Various aspects of the present invention will now be considered in order that its full scope can be appreciated.

### Diagnostics Uses

The p120/p100 band shift can be used to diagnose biopsy samples to report on the state of p120/p100 phosphorylation. Such diagnosis may be important in making therapeutic decisions or reporting on the efficacy of therapeutic drugs.

### Screening Uses

The p120/p100 band shift could be used as a method to screen for compounds which interfere with VEGF-initiated signalling pathways regulating p120/p100 phosphorylation. Identification of specific phosphorylated residues within p120 or p100 affected in response to VEGF could lead to the establishment of phosphatase or kinase assays and the screening of compounds affecting such enzymes. Appropriate fusion proteins or peptides could be used as substrates.

### Uses in Raising or Selecting Antibodies

A further use of the present invention is in raising or selecting antibodies. Antibodies recognizing p120 or p100 in phosphorylated or dephosphorylated forms could be raised and used as described under Diagnostic Uses and Screening Uses. Antibodies could be used in immunoblots, ELISAs or for immunocytochemistry of cultured cells or pathology specimens.

### Figures

The present invention will now be described by way of example only with reference to the accompanying figures; wherein:
Figure 1 shows an immunoblot of p120 and p100 and the effect of VEGF treatment, as described in Experiment 1 below;
Figure 2 shows an immunoblot of p120 and p100, which indicates that VEGF acts rapidly, as described in Experiment 2 below;
Figure 3 shows an immunoblot of p120 and p100, which indicates that VEGF acts potently, as described in Experiment 2 below;
Figure 4 shows an immunoblot of p120 and p100, which indicates that VEGF acts via VEGFR-2 (KDR/Flk-1), as described in Experiment 3 below;
Figure 5 shows an immunoblot, which indicates that the [³²P]phosphate incorporation into p120 and p100 and the results of probing the filter with antibody recognizing p120 and p100, as described in Experiment 4 below;
Figure 6 shows an immunoblot, which indicates that VEGF induces dephosphorylation of p 120 and p100 at similar sites, as described in Experiment 5 below;
Figure 7 shows an immunoblot, which indicates that VEGF stimulates serine/threonine dephosphorylation of p120 and p100 and does not affect tyrosine phosphorylation, as described in Experiment 6 below; and
Figure 8 shows that increased phosphotyrosine in response to VEGF does not colocalize with adherens junction associated protein, as described in Experiments 6 and 7 below.

### Methods

### Chemicals and Reagents

VEGF₁₆₅ was from PeproTech. Placenta growth factor (P/GF) was from R & D Systems. Phorbol-12,13-dibutyrate (PDB) was from Calbiochem-Novabiochem. Histamine was from Sigma Chemical Co. SU5416 was prepared by Tsukuba Research Laboratories, Eisai Co., Tsukuba, Japan. Collagen (Vitrogen 100) was obtained from Imperial Laboratories (Europe). [³²P]Phosphate (10 mCi/ml, carrier free) was from ICN-Flow Laboratories. *Staphyloccocus aureus* strain V8 protease (P-6306) was from Sigma.

### Antibodies

Mouse monoclonal antibodies recognising VE-cadherin (cadherin-5), N-cadherin, both p120 and p100, β-catenin and phosphotyrosine (PY20), and the rabbit polyclonal anti-phosphotyrosine, were from Transduction Laboratories. Peptide-directed antibodies recognizing α- and γ-catenin were provided by Kurt Herrenknecht (Eisai London Research Laboratories). Rabbit polyclonal antibody recognizing the activated forms (phosphoThr₂₀₂/phosphoTyr₂₀₄) of p42 and p44 MAP kinase was from New England BioLabs. Mouse monoclonal anti-vinculin (VIN-11-5) was from Sigma. Antibodies recognizing ZO-1 and CD31 were from Zymed and Dako, respectively. HRP-conjugated secondary antibodies were from Amersham. Both fluorochrome-conjugated (Cy3 or fluorescein) secondary antibodies and the rabbit anti-mouse IgG were from Jackson ImmunoResearch Labs.

### Cells and Treatments

Human umbilical vein endothelial cells (HUVEC), pooled donors, were from BioWhittaker and cultured in EGM on collagen-coated plastic (Falcon) according to the supplier's instructions. The cells were cultured in medium in the presence of 100 U/ml penicillin and 100 µg/ml streptomycin at 37°C in humidified air containing 10% CO₂. Routinely, stocks were maintained in 75 cm² flasks and split at a 1:3 ratio when almost confluent. For experimental use, cells were seeded in appropriate plasticware such that confluence was established within 2-3 days and the cells were usually used 5-6 days after seeding. Prior to experimental use, confluent monolayers of cells were equilibrated in fresh medium for at least 2 hours prior to the start of the experiment. Compounds and agents that were only soluble in Me₂SO were added to the culture medium of the cells in an equal volume of CO₂- and temperature-equilibrated medium containing twice the finally required concentration of compound. The final concentration of Me₂SO did not exceed 0.1% (v/v). Aqueous soluble agents were added at a 1:100 dilution directly to the cultures. In all cases, appropriate vehicle controls were performed and found to have no effect.

[³²P]Phosphate labelling was performed using Puck's Saline A (P-2917; Sigma) supplemented with 10 mM Hepes (Gibco), 2 mM glutamine (Gibco), 1 mM CaCl₂, 1 mM MgSO₄ and 0.2% (v/v) foetal calf serum. Cultures were rinsed twice with medium and then incubated with the same medium containing 100 µCi/ml of [³²P]phosphate. Labelling was for 2 hours at 37°C in humidified air with ambient CO₂.

### Protein Analysis

Whole cell lysates were prepared by aspiration of culture medium and immediately replacing it with boiling hot Laemmli sample buffer (Laemmli, U. K. (1970) Cleavage of structural proteins during the assembly of the head of bacteriophage T4. *Nature* **227**, 680-685) supplemented with 5 mM EDTA.Na₄. The samples were collected and heated at 100°C for 5 minutes. Proteins were resolved by gel electrophoresis using 8% acrylamide slab gels (Hoefer SE 600 system, 16 cm) and the buffers described (Laemmli, 1970, *loc cit*)*.* Gels were run overnight at 55 V. The gels were then equilibrated for 30 minutes in buffer at 4°C containing 48 mM Tris, 39 mM glycine, 0.03% SDS (w/v) and 20% methanol (v/v), and electrotransferred to nitrocellulose filters (Hybond ECL, Amersham) at 4°C. After Ponceau S staining, the filters were blocked in PBS containing 1% (w/v) BSA and 0.05% (v/v) Tween-20. The filters were then incubated with primary antibody, rinsed and immunoreactive bands were detected using appropriate HRP-conjugated secondary antibody and chemiluminescence (ECL, Amersham).

For analysis of the cadherin and its association with catenins, extraction and immunoprecipitations were performed at 4°C. Cultures were briefly rinsed with ice-cold PBS and then lysed in TX buffer (1% (v/v) Triton X-100, 25 mM Hepes, 2 mM EDTA, 0.1 M NaCl, 25 mM NaF, 1 mM vanadate, pH 7.6 (adjusted with NaOH), 1 mM PMSF, 10 µg/ml soybean trypsin inhibitor, 0.1 U/ml α₂-macroglobulin, 10 µg/ml leupeptin). The cells were extracted into lysis buffer for 10-15 minutes and then collected after scraping the dish. The lysates were centrifuged at 14,000 x *g* for 20 minutes. The supernatant was precleared with protein A-Sepharose (Pharmacia) for 1 hour and then incubated with primary antibody for 1 hour followed by a further 1 hour with protein A-Sepharose together with rabbit anti-mouse antibodies. After five washes in lysis buffer, immune complexes were dissociated by addition of Laemmli sample buffer followed by heating at 100°C for 5 minutes. Protein analysis was by SDS-PAGE and immunoblotting as described above.

For p120/p100-specific and phosphotyrosine (PY20) protein immunoprecipitations, the cells were lysed in boiling SDSSB (1% SDS, 25 mM Hepes, 2 mM EDTA, 0.1 M NaCl, 25 mM NaF, 1 mM vanadate, pH 7.6). The lysate was then collected, heated at 100°C for 5 minutes, cooled and diluted with four volumes of ice-cold TX buffer that contained 2.9% instead of 1% Triton X-100. Immunoprecipitation was performed as described for TX buffer extractions.

For phosphorylation analysis, cultures of HUVECs were labelled with [³²P]orthophosphate. After appropriate treatment, the cultures were lysed in boiling SDSSB and immunoprecipitated with the anti-p120/p100 antibody as described. For quantitation of [³²P]phosphate:protein, each immunoprecipitation was from protein extracted from ~10 cm² of confluent culture. For the phosphoaminoacid analysis and Cleveland maps, protein from ~60 cm² of confluent cells was used per immunoprecipitation. After separation by SDS-PAGE, for quantitation of [³²P]phosphate and p120/p100, protein was transferred to nitrocellulose and [³²P]phosphate incorporated into p120/p100 was detected by autoradiography. The filter was then probed with antibody recognizing p120 and p100 to quantitate protein levels, as described. For both the autoradiography and luminography, preflashed film was used. For phosphoamino acid analysis, after separation by SDS-PAGE, proteins were transferred to Immobilon P (Millipore). The area of filter containing p120 or p100 was detected by autoradiography, excised, and protein was acid hydrolysed to release phosphoaminoacids. These were then separated in two dimensions by high voltage electrophoresis at pH 1.9 and pH 3.5 and detected by autoradiography (Boyle et al., 1991). For proteolytic mapping (Cleveland, D. W., Fischer, S. G., Kirschner, M. W. and Laemmli, U. K. (1977) Peptide mapping by limited proteolysis in sodium dodecyl sulfate and analysis by gel electrophoresis. *J*. *Biol. Chem.* **252**, 1102-1106), the immunoprecipitated proteins were resolved by SDS-PAGE, briefly fixed and detected by autoradiography of the dried gels. Regions of gel containing p120 and p100 were excised. These gel slices were then rehydrated in TSE buffer (0.5 M Tris/HCl, pH 6.8; 0.1% SDS; 1 mM EDTA.Na₄) containing 20% glycerol, inserted into the well of the stacking gel containing 1 mM EDTA.Na₄ and overlaid with TSE buffer containing 10% glycerol and 100 ng of V8 protease. Following overnight electrophoresis at 60 V, the 15% gel, which again contained EDTA, was fixed, dried and autoradiographed at -80°C.

### Immunocytochemistry

Confluent monolayers of cells were fixed at room temperature for 15 minutes in 3% paraformaldehyde made up in PBS. Fixed cells were rinsed and then permeabilized by incubation with 0.5% Triton X-100 in PBS for 30 minutes. All antibodies were diluted in PBS containing 10% FCS, 0.1 M lysine, 0.3% Triton-X-100 and 0.02% azide. Incubation with antibody recognizing junction-associated protein was at 4°C overnight followed by incubation with mouse or rabbit anti-phosphotyrosine antibody for 2 hours at room temperature. Secondary antibodies recognizing mouse and rabbit IgG conjugated to Cy3 or fluorescein, respectively, were applied together for 1 hour at room temperature. After rinsing, the samples were mounted under Citifluor and viewed with epifluorescence on a Nikon Eclipse E800 microscope. Photographs were taken using Kodak T-MAX film (400 ASA).

### Experiment 1

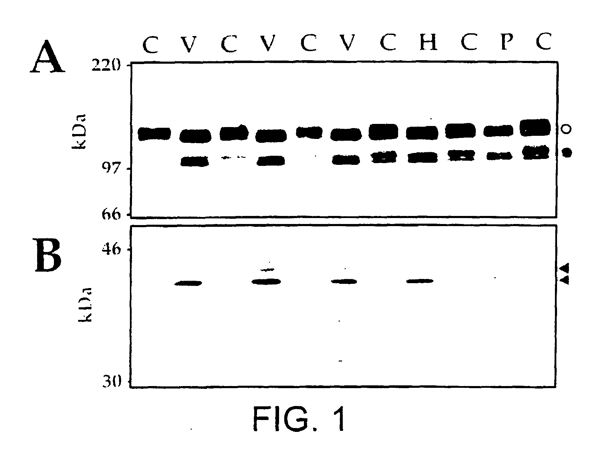

Human umbilical vein endothelial cells were incubated in the absence (Control - lane labelled C) or presence of 2.6 nM VEGF for 5 minutes. Incubations were performed in triplicate and analyzed in parallel. The cells were extracted in Laemmli sample buffer and proteins were resolved by SDS-PAGE. Following transfer to nitrocellulose, the filters were probed with antibody recognizing p120 and p100 (see Fig. 1A). p120 (migration indicated by the open circle) and p100 (migration indicated by the closed circle) from untreated cells migrate as broad bands. Those from VEGF treated cells (lane labelled V) migrate as faster, tighter bands. Other experiments have shown that this is due to the loss of phosphate from the protein on serine and threonine residues. For comparative purposes, the cells were also incubated with either 10 µM histamine (lane labelled H) or 200 nM phorbol 12,13 dibutyrate (lane labelled P) for 5 minutes and analyzed in parallel.

To reveal other changes in VEGF-stimulated signalling processes, the filters were also probed with an antibody recognizing the activated forms of p42 and p44 MAP kinase. VEGF clearly stimulated an increase in the amount of phosphorylated MAP kinase in parallel with its ability to affect p120/p100 migration (Fig. 1B). Histamine (H), likewise, increased the amounts of phospho-MAP kinases but the phorbol ester (P) was without effect (Fig. 1B).

All lanes received equal amounts of protein as determined by Ponceau S staining of the filters and verification by reprobing with antibody recognizing β-catenin (results not shown).

### Experiment 2

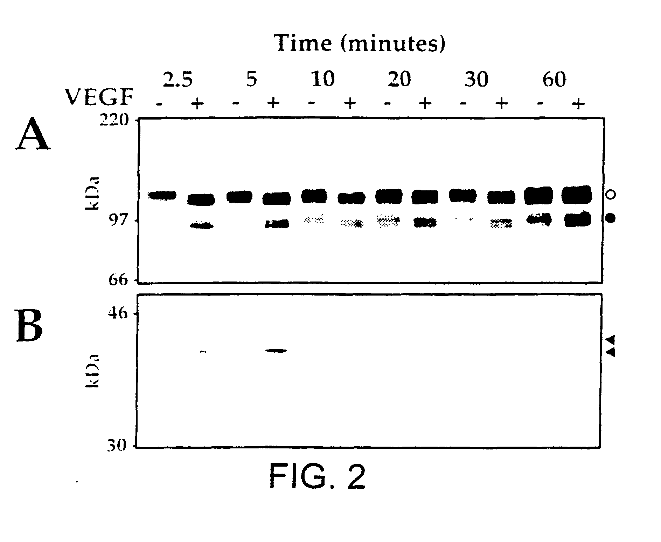

The kinetics and potency of the VEGF effect were examined. See Figure 2. Confluent monolayers of HUVECs were incubated in the absence (-) or presence (+) of 2.6 nM (100 ng/ml) VEGF for the times indicated. Cell extracts were prepared and p120 (O) and p100 (•) were detected by immunoblotting following SDS-PAGE, Figure 2, Panel A. The same filter was also probed with the antibody recognizing the activated forms of p42 and p44 MAP kinase (arrowheads), Figure 2, Panel B. Equal loading-of lanes was verified as in Fig. 1. VEGF acted very rapidly to trigger the increase in mobility of p120 and p100, effects were observed within 2 minutes and sustained for up to 30 minutes (Fig. 2A). After 60 minutes of incubation, the effect was less apparent (Fig. 2A). The kinetics paralleled those of the increase in amount of activated MAP kinase (Fig. 2B).

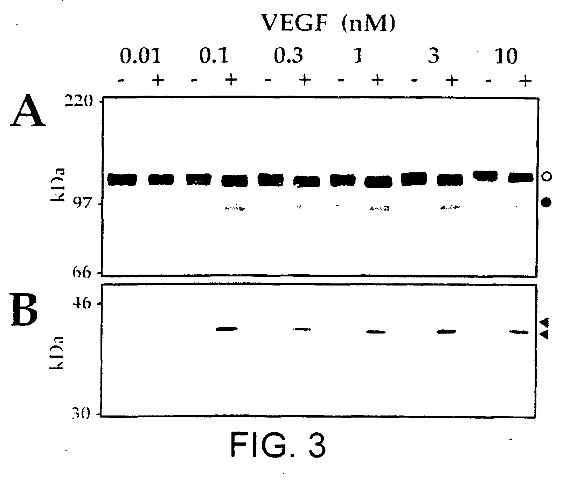

Confluent monolayers of HUVECs were incubated for 5 minutes in the absence (-) or presence (+) of the indicated concentrations of VEGF. Cell extracts were prepared and p120 (O) and p100 (•) were detected by immunoblotting following SDS-PAGE, Figure 3, Panel A. The same filter was also probed with the antibody recognizing the activated forms of p42 and p44 MAP kinase (arrowheads), Figure 3, Panel B. Equal loading lanes was verified as in Fig. 1. VEGF was also very potent at affecting p120/p100 mobility, effects were observed with concentrations as low as 0.1 nM (Fig. 3A). The sensitivity of the increase in amount of activated MAP kinase paralleled that of the increase in p120/p100 mobility (Fig. 3B).

### Experiment 3

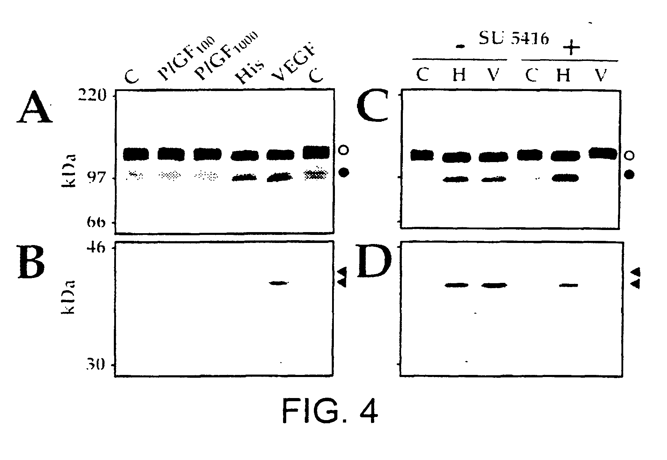

VEGF is known to bind to two receptors, VEGFR-1 and VEGFR-2. To determine which receptor(s) were responsible for the p120/p100 shift, the effects of placenta derived growth factor (P*l*GF), a VEGFR-1-specific ligand, were examined. Confluent monolayers of HUVECs were incubated for 5 minutes in the absence (lane labelled C) or presence of PIGF at 100 (P*l*GF₁₀₀) or 1000 ng/ml (P*l*GF₁₀₀₀). In parallel, the cells were treated with either 10 µM histamine (His) or 2.6 nM (100 ng/ml) VEGF. In another experiment, the cells were preincubated for 30 minutes in the absence (-) or presence(+) of 5 µM of the VEGFR-2 inhibitor SU5416. The cells were then treated for 5 minutes with either 2.6 nM VEGF (V) or 10 µM histamine (H). In all cases, cell extracts were prepared and p120 (O) and p100 (•) were detected by immunoblotting following SDS-PAGE, Figures 4A and 4C. The same filter was also probed with antibody recognizing the activated forms of p42 and p44 MAP kinase (arrowheads), Figures 4B and 4D. Equal loading of lanes was verified as in Fig. 1.

Even at concentrations up to 1000 ng/ml, PIGF failed to stimulate a mobility shift in p120/p100 (Fig. 4A) and also did not lead to activation of MAP kinase (Fig. 4B), whereas appropriate positive controls (VEGF, histamine) carried out in parallel were effective. These observations suggest that VEGF acted via VEGFR-2. This was corroborated by the finding that the VEGFR-2 specific inhibitor SU5416 (Sun, L., Tran, N., Tang, F., App, H., Hirth, P., McMahon, G. and Tang, C. (1998) Synthesis and biological evaluations of 3-substituted indolin-2-ones: a novel class of tyrosine kinase inhibitors that exhibit selectively toward particular receptor kinases. *J. Med. Chem.* **41**, 2588-2603. Fong, T. A. T., Shawver, L. K., Sun, L., Tang, C., App, H., Powell, J. P., Kim, Y. H., Schreck, R., Wang, X., Risau, W., Ullrich, A., Hirth, K. P. and McMahon, G. (1999) SU5416 is a potent and selective inhibitor of the vascular endothelial growth factor receptor (Flk-1/KDR) that inhibits tyrosine kinase catalysis, tumor vascularization, and growth of multiple tumor types. *Cancer Res.* **59** 99-106) blocked the ability of VEGF to stimulate an increase in p120/p100 mobility (Fig. 4C) and activation of MAP kinase (Fig. 4D). In contrast, SU5416 did not block the ability of histamine to stimulate a mobility shift in p120/p100 or to activate MAP kinase (Figs. 4C,D), indicating selectivity in its mode of action.

### Experiment 4

To explore the basis of the increase in mobility of p120/p100 stimulated by VEGF, phosphorylation analysis was performed. The cells were metabolically labelled with [³²P]phosphate, stimulated as required, lysed in buffer designed to preserve the phosphorylation state of proteins, immunoprecipitated with antibody recognizing p120 and p100 and then analyzed by SDS-PAGE, autoradiography and immunoblotting. This approach allows quantitation of phosphate per amount of protein.

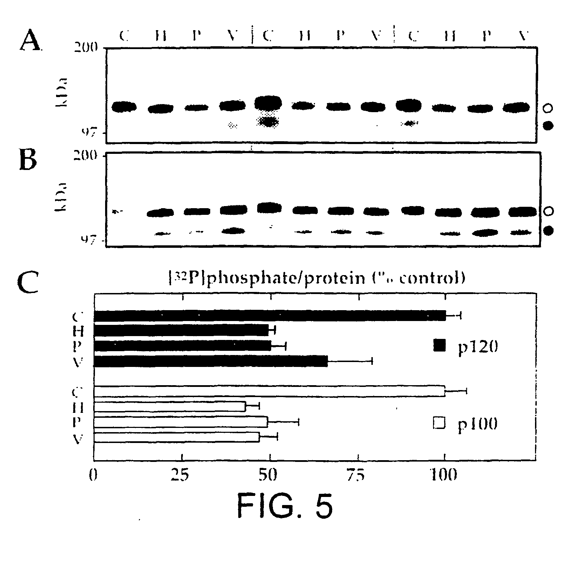

Confluent monolayers of HUVECs were metabolically labelled with [³²P]phosphate. The cells were then treated for 5 minutes with vehicle (lanes labelled C), 10 µM histamine (lanes labelled H), 200 nM PDB (lanes labelled P) or 2.6 nM VEGF (lanes labelled V). Following lysis in TDS buffer, p120 and p100 were immunoprecipitated, resolved by SDS-PAGE and transferred to nitrocellulose. An autoradiogram of the filter was taken to detect [³²P]phosphate incorporation into p120 (O) and p100 (•), Figure 5A, and the filter was then probed with antibody recognizing p120 and p100 to detect protein (Figure 5B). Autoradiography was for 40 hours at -80°C and the ECL exposure was taken in 1 minute. Band densities in the autoradiogram and luminogram were quantitated by densitometry and the [³²P]phosphate incorporation normalized to protein is expressed in Figure 5C. As a control, an aliquot of the supernatant from the immunoprecipitations was analyzed by SDS-PAGE and autoradiography. The result (not shown) indicated that there were no general effects on labelling of the multitude of proteins in this fraction.

p120 and p100 were detected as phosphoproteins in control cells and the [32P]phosphate:protein was determined (Fig. 5A). After incubation of the cells with VEGF, the autoradiogram showed that the [³²P]phosphate signal was less and the film density was similar to that of p120/p100 from control cells (Fig. 5A). Examination of the immunoblot of the same filter (Fig. 5B) showed that VEGF caused an increase in p120/p100 mobility and an increase in film density of the ECL signal. Quantitation by densitometry revealed that VEGF stimulated dephosphorylation of p120 and p100 (Fig. 5C), and that this dephosphorylation was similar in magnitude to that elicited by histamine or PDB (Figs. 5A-C). In response to VEGF, histamine and PDB; p120/p100 were -dephosphorylated resulting in increased electrophoretic mobility. The autoradiogram showed less of a signal whereas the immunoblot showed an increased intensity as the protein was concentrated during electrophoresis. In all cases, the supernatants from the immunoprecipitations were analyzed to confirm that there were no general effects on the phosphorylation of the many proteins present in these fractions (result not shown).

### Experiment 5

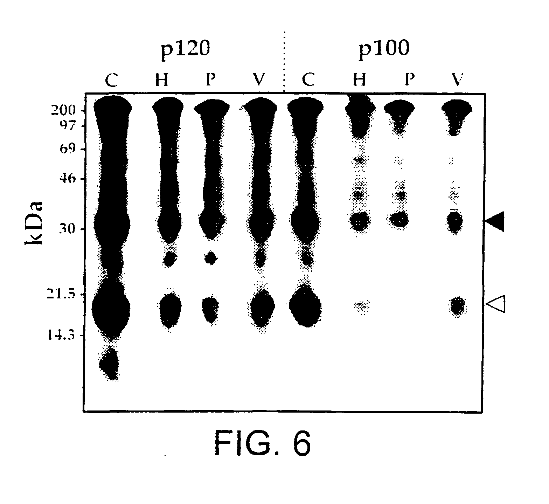

Phosphorylation sites within p120/p100 were analyzed. See Figure 6. HUVECs were metabolically labelled with [³²P]phosphate, stimulated as required and phosphorylation site analysis was performed by Cleveland mapping, a procedure involving limited proteolytic digestion.

Confluent monolayers of HUVECs were metabolically labelled with [³²P]phosphate. The cells were then treated for 5 minutes with vehicle (C), 10 µM histamine (H), 200 nM PDB (P) or 2.6 nM VEGF (V). Following lysis in TDS buffer, p120 and p100 were immunoprecipitated and resolved by SDS-PAGE. The gel was briefly fixed and an autoradiogram taken to localize bands corresponding to p120 and p100. These were excised from the gel partially digested with V8 protease and the digestion products were analyzed by SDS-PAGE. The labelling of digestion products was detected again by autoradiography. It was confirmed by immunoblotting that the amounts of p120 and p100 in the initial immunoprecipitates were equal.

For both p120 and p100, two major cleavage products at 30 kDa and 17 kDa were observed when protein was extracted from control cells (Fig. 6). After VEGF stimulation, phosphate incorporation into both of these fragments was substantially decreased (Fig. 6). After incubation of the cells with histamine or phorbol dibutyrate, similar effects on the p120 and p100 maps were observed (Fig. 6) suggesting that these different stimuli affect p120 and p100 phosphorylation at similar sites.

### Experiment 6

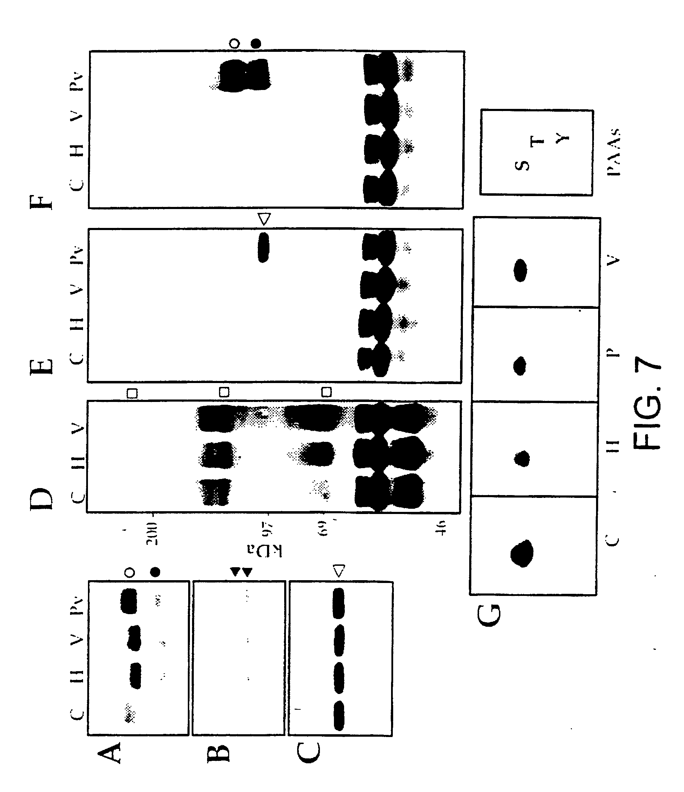

Two types of experiment were carried out to investigate which amino acids were phosphorylated. Confluent monolayers of HUVECs were incubated for 5 minutes in the absence (C) or presence of either 10µM histamine (H) or 2.6nM VEGF. Some cells were treated for 15 minutes with 100µM of the broad-spectrum tyrosine phosphatase inhibitor, pervanadate. Lysates of the cells were prepared by immediate extraction into a boiling solution of an SDS-based buffer containing kinase and phosphatase inhibitors, as described above. As positive controls for this experiment, the extracts were analyzed for a p120/p100 shift, activation of MAP kinase or content of β-catenin. As expected, VEGF and histamine produced effects on p120/p100 (Fig. 7A) and MAP kinase (Fig. 7B). Pervanadate (Pv) did not affect the mobility of p120/p100 (Fig. 7A) but did lead to activation of MAP kinase (Fig. 7B). The levels of βcatenin were found to be similar in all extracts (Fig. 7C).

These extracts were then immunoprecipitated with antiphosphotyrosine antibody PY20 and analyzed by immunoblotting with PY20 (Fig. 7D). In the controls, several major bands were observed. The open squares indicate the migration of bands corresponding, in order of decreasing size, to VEGFR-2, FAK and paxillin. The PY20 immunoprecipitates were also probed with antibody recognizing β-catenin (open arrow head), Panel E, p 120 (O) and p100 (•), Panel F.

The same PY20 immunoprecipitates were probed with antibodies recognizing β-catenin (Fig. 7E) or p120/p100 (Fig. 7F). Only after treatment with pervanadate were β-catenin and p120/p100 detected in the phosphotyrosine immunoprecipitates. A ten-times overexposure of the filters did not reveal any hint of β-catenin or p120/p100 in the phosphotyrosine immunoprecipitates in response to VEGF or histamine (results not shown). Therefore, even though the PY20 immunoprecipitates revealed tyrosine phosphorylation of proteins already reported to be tyrosine phosphorylated in response to VEGF and histamine, these same immunoprecipitates did not contain β-catenin or p120/p100.

Phosphoaminoacid analysis of p120/p100 was also performed. In control HUVECs, p120 and p100 were phosphorylated on mainly serine and, to a lesser extent, threonine residues (Fig. 7G). Confluent monolayers of HUVECs were metabolically labelled with [³²P]phosphate and received either vehicle (C), 10 µM histamine (H), 200 nM PDB (P) or 2.6 nM VEGF (V) for 5 minutes. Following lysis in TDS buffer and immunoprecipitation, phosphoaminoacid analysis of p120 and p100 was performed as described under Materials and Methods. The migration of phosphoaminoacid standards (PAAs) is also shown (S, phosphoserine; T, phosphothreonine; Y, phosphotyrosine). No phosphotyrosine was detected. After stimulation with VEGF, again phosphotyrosine was not detected but the amount of phosphoserine and phosphothreonine was decreased. Similar results were obtained after histamine and PDB treatment (Fig. 7G; previous experiments (Ratcliffe, M. J., Smales, C. S. and Staddon, J. M. (1999) Dephosphorylation of the catenins p120 and p100 in endothelial cells in response to inflammatory stimuli. *Biochem. J.* **338**, 471-478) were done with EA.hy926 cells). These observations indicated that p120 and p100 were dephosphorylated on serine and threonine residues in response to the various treatments.

Increased phosphotyrosine in response to VEGF does not colocalize with adherens junction associated protein. HUVECs were either treated with vehicle (Control) or 2.6 nM VEGF. After 5 minutes, the cells were fixed and then stained. In parallel, cells were also extracted and analyzed for p120/p100 by immunoblotting to confirm that VEGF had in fact stimulated a p120/p100 shift. Panel A shows the results of colabelling with mouse antibody recognizing β-catenin and rabbit antibody recognizing phosphotyrosine (pY). The localization of β-catenin was not affected by VEGF treatment. Although VEGF resulted in alteration of phosphotyrosine staining, this did not colocalize with the staining of β-catenin, which had a very distinctive fine structure. Panel B shows the results of colabelling with mouse antibody recognizing vinculin and rabbit antibody recognizing phosphotyrosine (pY). VEGF treatment resulted in the appearance of vinculin-positive striations close to regions of cell-cell contact; these striations colabelled with phosphotyrosine antibody. In all cases, it was confirmed that the phosphotyrosine staining was competed by phosphotyrosine but not by phosphoserine or phosphothreonine. All images were from similarly timed exposures. Bar, 20 µm.

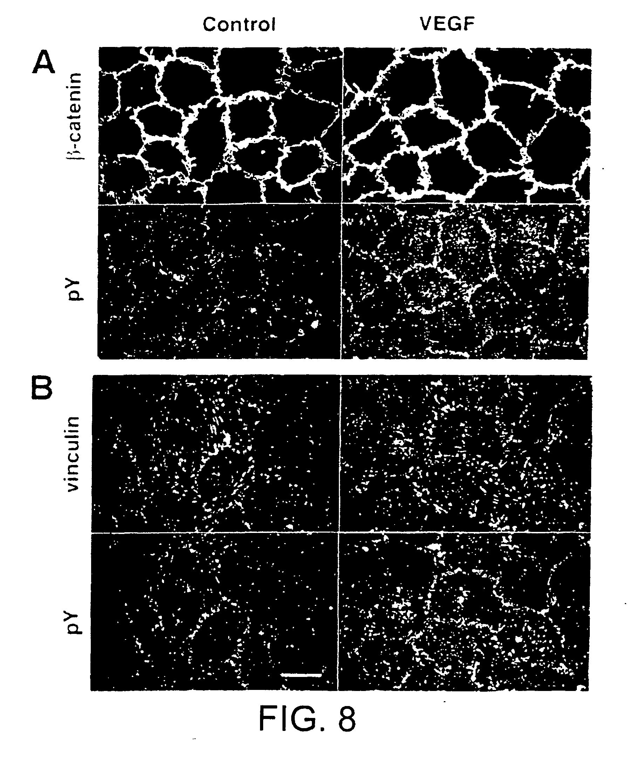

In control cells, protein phosphorylated on tyrosine was detected as striations within the cells (Figs. 8A,B). Following VEGF treatment, these striations increased and the cells could be outlined (Figs. 8A,B). Some of the phosphotyrosine in both the control and VEGF-treated cells colocalized with vinculin (Fig. 8B) but not β-catenin, which had a very distinctive fine structure (Fig. 8A).

These data agree with the immunoprecipitation analysis (Fig. 7D) where focal contact protein tyrosine phosphorylation was increased in response to VEGF but that of catenins, including β-catenin, was not. Similar experiments where the cells were colabelled with phosphotyrosine antibody and antibody recognizing either p120, CD31 or ZO-1, also failed to reveal colocalization with these other junctional proteins after addition of VEGF (results not shown).

### Experiment 7

Cadherins associate via their cytoplasmic tail with the catenins, including p120 and p100. To test if VEGF treatment resulted in altered association of catenins with cadherins in endothelial cells, the cells were lysed under detergent conditions designed to preserve association of the complex. Lysates from control or VEGF-stimulated (2.6 nM, 5 minutes) cells were immunoprecipitated with antibodies recognizing both p120 and p100 or β-catenin. The immunoprecipitates were analyzed by immunoblotting for the content of VE-cadherin, N-cadherin, α, β and γ catenin, p120 and p100. VEGF treatment, although increasing the electrophoretic mobility of p120 and p100, had no effect on association of any members of the complex (results not shown). Furthermore, immunocytochemical analysis also did not reveal any apparent effects on localization of β-catenin (Fig. 8) or p120/p100 (result not shown) to adherens junctions following VEGF treatment.

## Claims

1. The use of VEGF to screen *in vitro* for a substance capable of affecting the serine and/or threonine phosphorylation state of p120 and/or p100.

2. The use of VEGF, as claimed in claim 1, wherein the screen is for a substance capable of blocking the serine and/or threonine dephosphorylation of p120 and/or p100.

3. The use as claimed in claim 2, wherein the dephosphorylation is from the phosphorylated serine and/or threonine residues present in p120 and/or p100.

4. The use of a screen, as defined in any one of claims 1 to 3, to identify a substance which is capable of affecting the serine and/or threonine phosphorylation state of p120 and/or p100.

5. The use of VEGF to screen *in vitro* for a substance capable of interfering with a VEGF-initiated pathway regulating p120/p100 serine/threonine phosphorylation.

6. The use of VEGF, as claimed in claim 5, wherein the pathway is the PKC-p120/p100 pathway.

7. The use of VEGF, as claimed in 5 claim 6, wherein the screen is to identify an inhibitor and/or a competitor and/or an activator of VEGF.

8. A method to identify the serine and/or threonine phosphorylation state of p120 and/or p100 *in vitro* comprising the use of an antibody specific for one or more of the serine and/or threonine phosphorylation sites on p120 and/or p100 in the diagnosis of a disease involving VEGF.

9. A method to identify in vitro the serine and/or threonine phosphorylation state of p120 and/or p100 comprising the use of an antibody specific for one or more of the serine and/or threonine phosphorylation sites on p120 and/or p100 in the evaluation of efficacy of a drug being used or tested to control diseases involving VEGF.

10. A method to screen *in vitro* for a compound or other agent that interferes with a signalling pathway capable of being initiated by VEGF to regulate p120/p100 phosphorylation comprising the identification of the serine and/or threonine phosphorylation state of p120 and/or p100, in the presence of the compound or the other agent, and optionally comparing the phosphorylation state with a standard.

11. A method as claimed in claim 10, wherein the identification of the serine and/or threonine phosphorylation state is by monitoring a band shift of p120 and/or p100.

12. A method as claimed in claim 10 or 11, wherein the screen identifies a compound or other agent that interferes with a signalling pathway capable of being initiated by VEGF.

## Patentansprüche

1. Verwendung von VEGF zur in vitro Durchsuchung nach einer Substanz, die fähig ist, den Serin und/oder Threonin Phosphorylierungszustand von p120 und/oder p100 zu beeinflussen.

2. Verwendung von VEGF, gemäß Anspruch 1, wobei die Durchsuchung nach einer Substanz ist, die fähig ist, die Serin und/oder Threonin Dephosphorylierung von p 120 und/oder p 100 zu blockieren.

3. Verwendung gemäß Anspruch 2, wobei die Dephosphorylierung von den phosphorylierten Serin und/oder Threonin Resten ist, die in p120 und/oder p 100 anwesend sind.

4. Verwendung einer Durchsuchung, gemäß einem der Ansprüche 1 bis 3, zur Identifikation einer Substanz, welche fähig ist, den Serin und oder Threonin Phosphorylierungszustand von p120 und/oder p100 zu beeinflussen.

5. Verwendung von VEGF zur in vitro Durchsuchung nach einer Substanz, die fähig ist, mit
einem VEGF-initierten Weg, der die p120/p100 Serin/Threonin Phosphorylierung reguliert, zu interferieren.

6. Verwendung von VEGF, gemäß Anspruch 5, wobei der Weg der PKC-p1201p100 Weg ist.

7. Verwendung von VEGF, gemäß Anspruch 5 oder 6, wobei die Durchsuchung der Identifikation eines Inhibitors und/oder Kompetitors und/oder Aktivators von VEGF dient.

8. Verfahren zur Identifikation des Serin und/oder Threonin Phosphorylierungsstatus von p120 und/oder p100 in vitro, wobei das Verfahren, die Verwendung eines Antikörpers, welcher spezifisch für eine oder mehrere Phosphorylierungsstellen von Serin und/oder Threonin in p120 und/oder p100 ist, in der Diagnose einer Krankheit in der VEGF involviert ist, aufweist.

9. Verfahren zur Identifikation des Serin und/oder Threonin Phosphorylierungsstatus von p120 und/oder p100 in vitro, wobei das Verfahren, die Verwendung eines Antikörpers, welcher spezifisch für eine oder mehrere Phosphorylierungsstellen von Serin und/oder Threonin in p120 und/oder p100 ist, zur Evaluierung der Wirksamkeit eines Arzneimittels, welches zur Kontrolle von Krankheiten bei denen VEGF eingebunden ist, verwendet oder getestet wird.

10. Verfahren zur Durchsuchung in vitro, nach einer Verbindung oder anderen Agenzien, welche mit Signalwegen, die durch VEGF anregbar sind, um die Phosphorylierung von p120 und/oder p100 zu regulieren, interferieren, wobei das Verfahren die Identifikation des Serin und/oder Threonin Phosphorylierungsstatus in p120 und/oder p100, in der Gegenwart einer Verbindung oder anderen Agenzien aufweist, und gegebenenfalls Vergleichen des Phosphorylierungsstatus mit einem Standard.

11. Verfahren, gemäß Anspruch 10, wobei die Identifikation des Serin und/oder Threonin Phosphorylierungsstatus durch die Überwachung einer Bandenverschiebung von p120 und/oder p100 erfolgt.

12. Verfahren gemäß Anspruch 10 und 11, wobei die Durchsuchung eine Verbindung oder andere Agenzien identifiziert, welche mit Signalwegen interferieren, die durch VEGF anregbar sind.

## Revendications

1. Utilisation du VEGF pour cribler *in vitro* une substance pouvant affecter l'état de phosphorylation des sérines et/ou thréonines de p120 et/ou p100.

2. Utilisation du VEGF selon la revendication 1, dans laquelle on crible une substance pouvant bloquer la déphosphorylation des sérines et/ou thréonines de p120 et/ou p100.

3. Utilisation selon la revendication 2, dans laquelle la déphosphorylation a lieu à partir des résidus phosphorylés de sérine et/ou de thréonine présents dans p120 et/ou p100.

4. Utilisation d'un criblage selon l'une quelconque des revendications 1 à 3, pour identifier une substance qui peut affecter l'état de phosphorylation des sérines et/ou thréonines de p120 et/ou p100.

5. Utilisation du VEGF pour cribler *in vitro* une substance pouvant interférer avec une voie initiée par le VEGF qui régule la phosphorylation des sérine/thréonine des p120/p100.

6. Utilisation du VEGF selon la revendication 5, dans laquelle la voie est la voie PKC-p120/p100.

7. Utilisation du VEGF selon la revendication 5 ou la revendication 6, dans laquelle le criblage sert à identifier un inhibiteur et/ou un compétiteur et/ou un activateur du VEGF.

8. Procédé d'identification *in vitro* de l'état de phosphorylation des sérines et/ou thréonines de p120 et/ou p100, comprenant l'utilisation d'un anticorps spécifique à l'un ou à plusieurs des sites de phosphorylation sérine et/ou thréonine sur p120 et/ou p100, dans le diagnostic d'une maladie impliquant le VEGF.

9. Procédé d'identification *in vitro* de l'état de phosphorylation des sérines et/ou thréonines de p120 et/ou p100, comprenant l'utilisation d'un anticorps spécifique à l'un ou à plusieurs des sites de phosphorylation sérine et/ou thréonine sur p120 et/ou p100, dans l'évaluation de l'efficacité d'un médicament que l'on utilise ou que l'on évalue pour contrôler les maladies impliquant le VEGF.

10. Procédé de criblage *in vitro* d'un composé ou d'un autre agent qui interfère avec une voie de signalisation pouvant être initiée par le VEGF pour réguler la phosphorylation de p120/p100, comprenant l'identification de l'état de phosphorylation des sérines et/ou thréonines de p120 et/ou p100, en présence du composé ou de l'autre agent, et comprenant éventuellement la comparaison de l'état de phosphorylation avec un standard.

11. Procédé selon la revendication 10, dans lequel l'identification de l'état de phosphorylation des sérines et/ou thréonines se fait en contrôlant un déplacement des bandes de p120 et/ou p100.

12. Procédé selon la revendication 10 ou 11, dans lequel le criblage identifie un composé ou un autre agent qui interfère avec une voie de signalisation pouvant être initiée par le VEGF.
